(19)

Europäisches
Patentamt
European
Patent Office
Office européen
des brevets

(11) **EP 3 936 586 B1**

(12) **EUROPEAN PATENT SPECIFICATION**

(45) Date of publication and mention
of the grant of the patent:
**03.09.2025 Bulletin 2025/36**

(21) Application number: **20766237.0**

(22) Date of filing: **26.02.2020**

(51) International Patent Classification (IPC):
*C10B 57/04* (2006.01)      *G01N 33/22* (2006.01)
*G01N 13/02* (2006.01)

(52) Cooperative Patent Classification (CPC):
**G01N 13/02; C10B 57/04; G01N 33/222**

(86) International application number:
**PCT/JP2020/007700**

(87) International publication number:
**WO 2020/179576 (10.09.2020 Gazette 2020/37)**

(54) **EVALUATION METHOD FOR COAL, PREPARATION METHOD FOR BLENDED COAL, AND PRODUCTION METHOD FOR COKE**

VERFAHREN ZUR BEWERTUNG VON KOHLE, VERFAHREN ZUR HERSTELLUNG VON GEMISCHTER KOHLE UND VERFAHREN ZUR HERSTELLUNG VON KOKS

PROCÉDÉ D'ÉVALUATION DE CHARBON, PROCÉDÉ DE PRÉPARATION DE CHARBON MÉLANGÉ, ET PROCÉDÉ DE PRODUCTION DE COKE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.03.2019 JP 2019038505**

(43) Date of publication of application:
**12.01.2022 Bulletin 2022/02**

(73) Proprietor: **JFE Steel Corporation
Tokyo 100-0011 (JP)**

(72) Inventors:
• **IGAWA, Daisuke
Tokyo 100-0011 (JP)**
• **DOHI, Yusuke
Tokyo 100-0011 (JP)**
• **MATSUI, Takashi
Tokyo 100-0011 (JP)**

(74) Representative: **Grünecker Patent- und
Rechtsanwälte
PartG mbB
Leopoldstraße 4
80802 München (DE)**

(56) References cited:
**WO-A1-2013/145678      WO-A1-2013/145679
WO-A1-2013/145680      WO-A1-2014/129337
JP-A- 2015 193 829       JP-A- 2018 197 319
JP-A- 2019 031 641**

• **NAGAYAMA MIKIYA ET AL: "Evaluation of Coal
Compatibility Effect in Coke Strength by Surface
Tension of Semi-coke", vol. 57, no. 6, 1 January
2017 (2017-01-01), JP, pages 989 - 995,
XP055895517, ISSN: 0915-1559, Retrieved from
the Internet <URL:https://www.jstage.jst.go.jp/
article/isijinternational/57/6/57_ISIJINT-2016-660/
_pdf/-char/en> [retrieved on 20220225], DOI:
10.2355/isijinternational.ISIJINT-2016-660**

**Description**

Technical Field

The present invention relates to a method for evaluating coal that serves as a raw material for metallurgical coke and to methods for preparing a coal blend that use measured values used in the method for evaluating coal. The present invention further relates to a method for producing coke from a coal blend prepared by the method for preparing a coal blend.

Background Art

It is desirable that metallurgical coke, which is used as a blast furnace raw material for producing pig-iron in a blast furnace, have high strength. If coke has low strength, the coke is degraded in a blast furnace, and the degraded coke impairs the permeability of the blast furnace; consequently, pig-iron cannot be produced consistently.

Typically, coke is produced by carbonizing a coal blend, which is prepared by blending together two or more types of coal, in a coke oven. Various methods are known as methods for blending coal for obtaining coke having a desired strength.

Patent Literature 1 discloses a method for blending coal, which is a method that takes coal compatibility into account. In the method, by using, as an index, the value of an interfacial tension calculated from surface tensions of semi-coke, which is obtained by heat treating each of the brands of coal that constitute a coal blend, and a blending ratio (mass fraction) of each of the brands of coal in the coal blend, the blending ratio of the coal is adjusted. As used herein, the term "coal compatibility" refers to a property in which the plural brands of coal in a coal blend interact with one another. It is known that in some instances, depending on the coal compatibility, an additive property is not valid for the strengths of coke derived from the respective types of coal of a coal blend and the strength of coke derived from the coal blend.

A further method for evaluating coal is disclosed by Nagayama Mikiya ET AL: "Evaluation of Coal Compatibility Effect in Coke Strength by Surface Tension of Semi-coke", ISIJ INTERNATIONAL, Vol. 57 (2017), No. 6, pages 989-995.

Citation List

Patent Literature

PTL 1: International Publication No. 2013/145680

Non Patent Literature

NPL 1: D.W. Fuerstenau: International Journal of Mineral Processing, 20 (1987), 153

Summary of Invention

Technical Problem

In recent years, from the standpoint of ensuring consistent procurement of coal resources and reducing the raw material cost, it has been increasingly necessary to purchase coal mined at more than one location and use the plural brands of coal having different properties, as raw materials of a coal blend. In instances where several types of coal having different properties are to be used in a coal blend, the method disclosed in Patent Literature 1 can be used to prepare a coal blend from which coke having a desired strength is expected to be produced. However, regarding some types of coal, there have been instances in which coke not having a desired strength was produced even in a case in which the coal blend was prepared by blending together plural brands of coal in mass fractions determined by the method disclosed in Patent Literature 1.

The present invention was completed in view of the instances described above. By implementing a method of the present invention for preparing a coal blend, types of coal that are to constitute a coal blend from which coke having a desired strength can be produced and mass fractions of the types of coal can be accurately specified compared with related-art techniques. An object of the present invention is to provide such methods for preparing a coal blend. In addition to the methods for preparing a coal blend, the present invention also provides a method for producing coke that uses the preparation method and provides a method for evaluating coal that serves as a raw material for metallurgical coke.

Solution to Problem

The present inventors identified the following phenomenon: in instances where an inert amount in coal was varied, a variation in surface tensions of semi-coke, which was obtained by heat treating the coal, relative to the amount by which the inert amount was varied (variation), varied between different brands of coal. The present inventors studied the phenomenon and first completed a method for evaluating coal based on the inert amounts of coal and the surface tensions of semi-coke.

For example, in the related-art method described in Patent Literature 1, the surface tension of semi-coke obtained by heat treating one brand of coal is given as a mean value in a distribution of measured surface tensions. In the knowledge of the related art, it was merely inferred that a reason that the distribution of surface tensions occurs is inhomogeneity of coal, and details have not been elucidated. The present inventors performed more detailed studies regarding reasons for the variance in the surface tension value of semi-coke associated with the components of coal. Components of coal that particularly affect coke strength are a component that softens and melts when heated (the component may be hereinafter referred to as "reactive") and a component that does not soften or melt even when heated (the component may be hereinafter referred to as "inert"). Based on this fact, the present inventors performed studies to develop a method for estimating surface tension values of semi-coke resulting from heat treatment of the respective two components.

No methods are known for, regarding the components in coal, accurately separating a component that softens and melts when heated from a component that does not soften or melt when heated; however, it is known that the inert, which can be identified by observing the coal with a microscope, generally corresponds to the component in coal that does not soften or melt. A method for analyzing a content of the inert is specified in JIS M 8816. The present inventors employed the total inert (TI) specified in JIS M 8816 as a content of the component that does not soften or melt when heated.

It is difficult to separate the component of coal that softens and melts from the component thereof that does not soften or melt. For an alternative means for estimating the surface tensions of semi-coke derived from the respective components, the present inventors focused their attention on the inert. The present inventors made the following attempt: separating coal into a portion having a high inert content and a portion having a low inert content (an inert content may be hereinafter referred to as an "inert amount"); heat treating the respective portions to form semi-coke; and, from the surface tension values of the semi-coke, estimating the surface tension value of coal having a 100% inert content and the surface tension value of coal having a 0% inert content.

The inert of coal is harder than the reactive thereof. This fact is utilized for the separation of coal into a portion having a high inert amount and a portion having a low inert amount. Since inert is harder than reactive, when coal is pulverized, inert tends to be concentrated in coarse-side coal particles. By utilizing this tendency, samples having different inert amounts can be prepared from the same brand of coal, by pulverization and sieving. Semi-coke was prepared by heat treating such samples, and surface tensions thereof were measured. As a result, the present inventors discovered that the variation in the surface tensions relative to the variation in the inert amounts varies between different brands of coal; and, in a case where samples are obtained by separating coal into a portion having a high inert amount and a portion having a low inert amount, there is a generally linear relationship between the inert amounts of the samples and the surface tensions of semi-coke obtained by heat treating the samples. In addition, the present inventors discovered that since the ratio between the variation in the inert amounts and the variation in the surface tensions affects the strength of coke that is derived from a coal blend that includes the brand of coal, evaluations as to whether the brand of coal is favorable as a raw material for coke can be made based on the ratio between the variations.

The present inventors completed the methods for preparing a coal blend by using items identified in the evaluation described above. In the methods for preparing a coal blend of the present invention, an assumption is made that each of the brands of (one brand of) coal in a coal blend is formed of inert, in which a proportion of an inert component is 100%, and reactive, in which a proportion of the inert component is 0%; by using a regression line, which is based on the inert amounts of coal and the surface tensions of semi-coke obtained by heat treating the samples as determined by the evaluation method described above, a surface tension $\gamma_{100}$, which corresponds to an inert content of 100%, and a surface tension $\gamma_0$, which corresponds to an inert content of 0%, are determined; and an assumption is made that a surface tension of semi-coke derived from the inert is equal to $\gamma_{100}$, and a surface tension of semi-coke derived from the reactive is equal to $\gamma_0$. In methods for preparing a coal blend of the present invention, based on the assumption, an interfacial tension is calculated from a blending ratio, a mass fraction of the inert, a mass fraction of the reactive, the surface tension $\gamma_{100}$, and the surface tension $\gamma_0$ of each of the brands of coal of a coal blend; and, based on a relationship between the interfacial tension and a strength of coke produced from the coal blend, mass fractions of the respective brands are specified. It is further possible to specify mass fractions of the respective brands of the coal blend such that the blending ratio of coal having a high ratio of the variation in the surface tensions of semi-coke to the variation in the inert amounts is limited.

Specifically, a summary of the present invention is as specified in the appended claims.

Advantageous Effects of Invention

With the present invention, types of coal that are to constitute a coal blend from which coke having a desired strength can be produced and mass fractions of the types of coal can be accurately specified compared with related-art techniques. Accordingly, it is possible to prepare a coal blend from which coke having a desired strength can be produced. Furthermore, with the present invention, it is possible to evaluate coal that serves as a raw material for metallurgical coke.

Brief Description of Drawings

[Fig. 1] Fig. 1 is a graph illustrating a relationship between an interfacial tension, which was calculated by the method described in Patent Literature 1, and a strength of coke.
[Fig. 2] Fig. 2 is a graph illustrating a relationship between inert amounts of coal samples and surface tensions of semi-coke obtained by heat treating the coal samples.
[Fig. 3] Fig. 3 is a graph illustrating a relationship between an interfacial tension, which was calculated by a method of the present invention, and the strength of coke.
[Fig. 4] Fig. 4 is a graph illustrating a relationship between the strength of coke and a blending ratio of low-value coal.

Description of Embodiments

The present invention relates to a method for evaluating coal, and the method uses surface tensions of semi-coke obtained by heat treating coal. The present invention also relates to methods for preparing a coal blend that includes at least two brands of coal, and the methods utilize items identified by the evaluation method. The method for evaluating coal of the present embodiment utilizes surface tensions of semi-coke and an interfacial tension calculated from the surface tensions. Accordingly, first, a method for preparing the semi-coke, a method for measuring the surface tension of the semi-coke, and a method for calculating the interfacial tension will be described.

[Method for Preparing Semi-coke]

Semi-coke is a heat-treated product obtained by heat treating coal. Adhesive phenomena of coal affect the compatibility between different types of coal and affect a strength of coke. Accordingly, in instances where the adhesive phenomena of coal is to be studied, it is preferable to determine properties of a coal melt at a temperature of 350 to 800°C, within which the coal is heated, the coal actually begins to soften and melt, and the coal adheres and solidifies for the completion of coking. However, no methods are known for measuring properties of a coal melt in such a high temperature range. In instances where the property to be measured is a surface tension, the surface tension value of coal in a plastic state can be estimated by, as described in Patent Literature **1,** measuring surface tensions of semi-coke, which is obtained by carbonizing the coal by heating the coal to a temperature at which the coal softens and melts and thereafter performing cooling.

Regarding a heating temperature for heating coal, the heating is to be carried out at a temperature of greater than or equal to 350°C, at which coal begins to soften and melt, to 800°C, at which coking is completed, as described above. This is appropriate from the standpoint that the surface tension has an influence on adhesion between coal particles. In the heating temperatures of 350 to 800°C, the temperatures that particularly contribute to adhesion are temperatures of 350 to 550°C, which are temperatures at which softening and melting occurs, and it is believed that an adhesion structure is determined at approximately 500°C. Accordingly, it is preferable that the heating temperature be 480 to 520°C, in the vicinity of 500°C. It is preferable that the heating be performed in an atmosphere of an inert gas (e.g., nitrogen, argon, helium, or the like) that is not reactive with coal.

It is preferable that the cooling be performed in an atmosphere of an inert gas that is not reactive with coal. Furthermore, it is preferable that the carbonized coal be quenched at a cooling rate of 10°C/second or greater. A reason for the quenching is to maintain the molecular structure achieved in the plastic state, and, therefore, it is preferable that the cooling be performed at a cooling rate of 10°C/second or greater, at which it is believed that the molecular structure does not change. Methods for the quenching include methods that use liquid nitrogen, ice water, water, or an inert gas, such as nitrogen gas. It is preferable that the quenching be performed by using liquid nitrogen. Gas cooling is not preferable because, with such cooling, cooling the inside of coal takes time, and a variation in the cooling rate is significant. Cooling with ice water or water is not preferable because, with such cooling, deposition of moisture affects the measurement of the surface tension.

In the present invention, exemplary operations of the heat treatment performed on coal are as described in (a) to (c) below.

(a) Coal is pulverized. Regarding a pulverized particle size of the coal, from the standpoint of preparing a homogeneous sample from coal, which is inhomogeneous in terms of constituents, properties, and the like, it is preferable that the coal be pulverized to a particle size of less than or equal to 250 $\mu$m, which is a particle size for the proximate analysis of coal described in JIS M 8812. More preferably, the coal is pulverized to a smaller particle size of less than or equal to 200 $\mu$m.

(b) The coal pulverized in operation (a) is heated at an appropriate heating rate in a state in which air is blocked or in an inert gas atmosphere. It is sufficient that the coal be heated to a temperature within the above-mentioned range of 350 to 800°C. It is preferable that the heating rate be a rate in accordance with a heating rate used in the production of coke in a coke oven.

(c) The coal heated in operation (b) is cooled. It is preferable that the cooling be carried out in the manner described above.

[Method for Measuring Surface Tension of Semi-coke]

Known methods for measuring a surface tension of a common substance include sessile drop methods, capillary-rise methods, maximum bubble pressure methods, drop weight methods, pendant drop methods, ring methods, plate (Wilhelmy) methods, advancing/receding contact angle methods, tilting plate methods, and the like. Since coal is formed of various molecular structures, and the surface tensions thereof are not uniform, the measurement of the surface tension of coal may be performed by using a method that can evaluate a surface tension distribution, and an example of the method is the Film Flotation method described in Non-Patent Literature 1. This method can be used for both coal and semi-coke derived from the coal, in a similar manner, and a distribution of surface tensions of coal can be determined by using a finely pulverized coal sample. A mean value in the obtained distribution of surface tensions is designated as a representative value of the surface tensions of the coal sample. In instances where semi-coke is used as a sample, it is preferable that a heat treatment temperature for heat treating coal be set to be within a thermoplastic temperature range of coal. Details of the measurement method are described in Patent Literature 1.

[Method for Calculating Interfacial Tension]

A method for calculating the interfacial tension is as follows. With attention focused on two brands of coal out of the plural brands of coal included in a coal blend, the method includes a step of determining an interfacial tension $\gamma_{ij}$, which is an interfacial tension between two types of semi-coke derived from the respective two brands of coal, and a step of calculating an interfacial tension $\gamma_{blend}$, which is an interfacial tension of the coal blend, from the interfacial tension $\gamma_{ij}$ and the mass fraction of each of the brands of coal in the coal blend. Since the interfacial tension $\gamma_{blend}$ of the coal blend is calculated based on the interfacial tension between different types of semi-coke, the interfacial tension $\gamma_{blend}$ can be regarded as a value corresponding to an interfacial tension of semi-coke derived from the coal blend; however, in the present invention, the interfacial tension determined in the manner described is referred to as the interfacial tension $\gamma_{blend}$ of a coal blend.

Now, a step for determining the interfacial tension $\gamma_{ij}$ between two brands of semi-coke will be described. For two types of common substances, the interfacial tension thereof can be directly measured, or the value of the interfacial tension can be determined from surface tensions of the respective substances. For example, regarding different substances i and j, the interfacial tension $\gamma_{ij}$ between the substance i and the substance j can be determined from a surface tension $\gamma_i$ of the substance i and a surface tension $\gamma_j$ of the substance j. The interfacial tension $\gamma_{ij}$ between the substance i and the substance j can be represented by equation [1] below, which is the Girifalco-Good equation.

[Math. 1]

$$\gamma_{ij} = \gamma_i + \gamma_j - 2\phi\sqrt{\gamma_i\gamma_j} \qquad\qquad [1]$$

In equation [1], $\varphi$ is an interaction parameter; the interaction parameter $\varphi$ can be determined experimentally and is known to vary depending on the substances i and j. Furthermore, D. Li and A. W. Neumann et al. assumed that the value of the interaction parameter $\varphi$ increases as the difference between the value of the surface tension $\gamma_i$ of the substance i and the value of the surface tension $\gamma_j$ of the substance j increases and, accordingly, proposed equation [2] below, which is an equation extended from equation [1].

[Math. 2]

$$\gamma_{ij} = \gamma_i + \gamma_j - 2exp\left[-\beta(\gamma_i - \gamma_j)^2\right]\sqrt{\gamma_i\gamma_j} \qquad\qquad [2]$$

In equation [2], $\beta$ is an experimentally derived constant. D. Li and A. W. Neumann et al. performed calculations and found that $\beta$ is 0.0001247 $(m^2/mJ)^2$. Assuming that the substance i is semi-coke i derived from coal i, and the substance j is semi-coke j derived from coal j, the interfacial tension $\gamma_{ij}$ between the semi-coke i and the semi-coke j can be calculated by measuring the surface tension $\gamma_i$ of the semi-coke i and the surface tension $\gamma_j$ of the semi-coke j and substituting the values of the surface tensions into equation [1] or equation [2]. In instances where equation [1] is used, the value of the interaction parameter $\varphi$ needs to be determined experimentally. Accordingly, in terms of simplifying the calculation of the interfacial tension, it is preferable to use equation [2], which uses the estimated value of the interaction parameter $\varphi$.

Now, a step for calculating the interfacial tension $\gamma_{blend}$ of a coal blend will be described. In instances where the brands of coal in a coal blend and the mass fractions thereof are known, the interfacial tension of semi-coke derived from the coal blend can be calculated. Assuming that, in an instance where n brands of coal are present in a coal blend, the mass fractions thereof are denoted as $w_i$ (which represents the mass fractions of 1st, 2nd,...ith,...and nth coal), the probability of presence of i-j interfaces formed between semi-coke derived from coal i and semi-coke derived from coal j can be represented by the product of $w_i$ and $w_j$. When the interfacial tension at the interfaces of the semi-coke is designated as $\gamma_{ij}$, the interfacial tension $\gamma_{blend}$, which is an interfacial tension of semi-coke derived from a coal blend in which the n brands of coal are blended, can be expressed by the following equation [3].

[Math. 3]

$$\gamma_{blend} = \sum_{i=1}^{n} \sum_{j=1}^{n} w_i w_j \gamma_{ij} \qquad [3]$$

In equation [3], in reality, it is preferable that $w_i$ and $w_j$ be each expressed as a mass fraction of the semi-coke in a semi-coke mixture derived from the coal blend. However, abundance ratios of types of semi-coke, which are derived from the respective types of coal, in the semi-coke mixture, are not significantly different from abundance ratios of the respective types of coal in the coal blend. Accordingly, $w_i$ and $w_j$ are each expressed as the mass fraction of the coal in the coal blend.

In the present invention, an assumption is made that several types of semi-coke derived from respective types of coal and constituents thereof that constitute a coal blend are mixed together, and the interfacial tension $\gamma_{blend}$ corresponds to an interfacial tension of the semi-coke mixture, which is assumed to result from the mixing. That is, the present invention is not predicated on the actual production of a semi-coke mixture, and the interfacial tension of the present invention is an index (value) determined from surface tensions of semi-coke and the mass fractions of coal, from which the semi-coke is derived, in a coal blend.

Now, a comparative experiment will be described, which was conducted to determine a relationship between an interfacial tension $\gamma_{blend}$ and a coke strength DI150/15 of coke produced from a coal blend obtained by blending plural brands of coal together; the interfacial tension $\gamma_{blend}$ was calculated by using the method disclosed in Patent Literature 1, for comparison with the present invention.

<Comparative Experiment>

A plurality of coal blends, each of which was formed of plural brands of coal, were prepared, and the coal blends were carbonized by using an electric furnace that can simulate the carbonization conditions of a coke oven. Accordingly, coke was produced.

Surface tensions of semi-coke obtained by heat treating, at 500°C, each of the brands of coal that constitute the coal blend were measured, and the interfacial tension $\gamma_{blend}$ was calculated by using equation [2] and equation [3]. Furthermore, a drum strength DI(150/15) was employed as the strength of coke. Specifically, in accordance with the test method for Drum strength of coke of JIS K 2151, a drum testing machine charged with a predetermined amount of coke was rotated at 15 rpm 150 times, and subsequently, the mass fraction of coke having a particle diameter of 15 mm or greater was measured. The drum strength DI(150/15) is the mass ratio of the mass fraction relative to the mass fraction before the rotation.

The coal used in the comparative experiment and properties thereof are shown in Table 1.

[Table 1]

| Coal | log MF (log/ddpm) | Ro (%) | TI (%) | Volatile matter (d.b) | Ash (d.b) | Surface tension y (mN/m) |
|------|-------------------|--------|--------|-----------------------|-----------|--------------------------|
| A | 0.90 | 1.59 | 23.6 | 17.5 | 9.3 | 40.3 |
| B | 1.23 | 1.49 | 18.7 | 18.1 | 8.6 | 40.1 |
| C | 0.48 | 1.56 | 21.0 | 17.1 | 10.8 | 39.1 |

(continued)

| Coal | log MF (log/ddpm) | Ro (%) | TI (%) | Volatile matter (d.b) | Ash (d.b) | Surface tension y (mN/m) |
|------|-------------------|--------|--------|------------------------|-----------|---------------------------|
| R | 2.97 | 1.20 | 20.4 | 24.2 | 10.8 | 40.5 |

In the comparative experiment, A to C and R, as shown in Table 1, were prepared, and coals A to C and R were blended together in the mass fractions shown in Table 2. Accordingly, coal blends 1 to 4 were prepared.

[Table 2]

|  | Coal blend 1 | Coal blend 2 | Coal blend 3 | Coal blend 4 |
|--|--------------|--------------|--------------|--------------|
| Mass fraction (%) of coal A | 50 | - | - | 30 |
| Mass fraction (%) of coal B | - | 50 | - | 20 |
| Mass fraction (%) of coal C | - |  | 50 | - |
| Mass fraction (%) of coal R | 50 | 50 | 50 | 50 |

| log MF (log/ddpm) | 1.94 | 2.10 | 1.72 | 2.00 |
|-------------------|------|------|------|------|
| Ro (%) | 1.39 | 1.34 | 1.38 | 1.37 |
| TI (%) | 22.0 | 19.5 | 20.7 | 21.0 |
| $\gamma_{blend}$ (mN/m) | 0.0003 | 0.0013 | 0.0159 | 0.0008 |
| DI150/15 (-) | 82.0 | 82.6 | 81.0 | 82.5 |

In Table 1, "Surface tension y (mN/m)" is a surface tension of semi-coke obtained by heat treating each of coals A to C and R and is a mean value in a surface tension distribution measured by using the Film Flotation method. In Table 1 and Table 2, "Ro (%)" is the mean maximum reflectance of vitrinite in coal (a coal blend) according to JIS M 8816, and "TI (%)" is an inert amount (vol%) for analysis of coal macerals calculated by using the method for measuring macerals of coal (a coal blend) of JIS M 8816 and equation [4] below, which is based on the Parr Equation described in an explanation of the method.

Inert amount (vol%) = fusinite (vol%) + micrinite (vol%) + (2/3)×semifusinite (vol%) + mineral matter (vol%)          [4]

In Tables 1 and 2, "log MF" is the common logarithm of a maximum fluidity (MF) of coal or a coal blend as measured by the Gieseler plastometer method described in JIS M 8801. The maximum fluidity log MF of a coal blend is a weighted average of the logs MF of the respective brands of coal in the coal blend. In "Ash (d.b)" and "Volatile matter (d.b)" in Table 1, the values are measured dry base values determined by a method for proximate analysis described in JIS M 8812.

In Table 2, $\gamma_{blend}$ represents the interfacial tension of coal blends 1 to 4 calculated from the value of the surface tension $\gamma$ shown in Table 1 and the mass fractions shown in Table 2, in accordance with equation [2] and equation [3]. "DI150/15 (-)" is the strength of coke obtained by carbonizing coal blends 1 to 4.

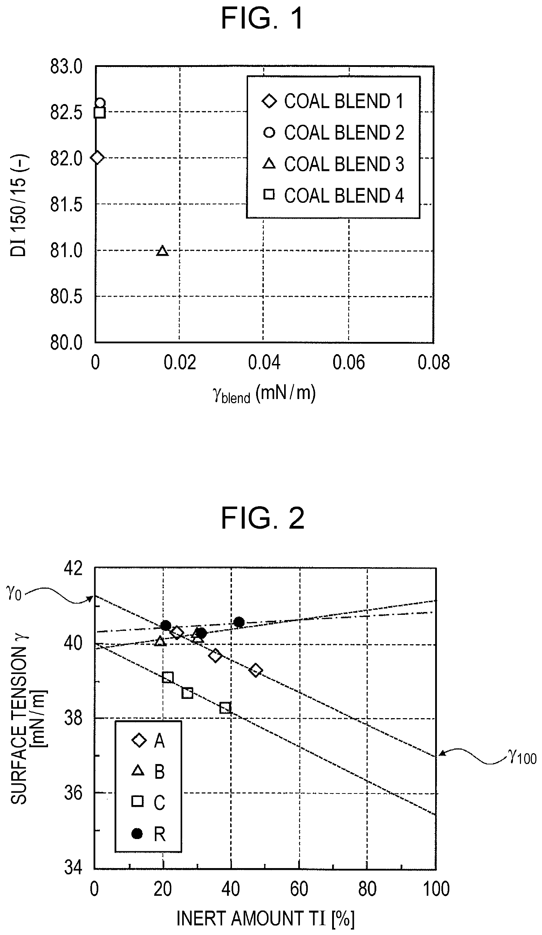

Fig. 1 is a graph illustrating a relationship between an interfacial tension $\gamma_{blend}$, which was calculated by the method described in Patent Literature 1, and the coke strength DI150/15 (-). As can be seen from Fig. 1, in coal blends 1, 2, and 4, the values of the interfacial tensions $\gamma_{blend}$ were close to 0, but there were variations in the coke strength DI150/15. In coal blend 3, the values of the interfacial tension $\gamma_{blend}$ and the coke strength DI150/15 exhibited a tendency different from those of coal blends 1, 2, and 4.

Patent Literature 1 states that a highly correlative relationship holds between the interfacial tension $\gamma_{blend}$ and the strength DI150/15. In some instances, a tendency of a highly correlative relationship is observed between the interfacial tension $\gamma_{blend}$ and the strength DI150/15, whereas in other instances, as shown in Fig. 1, the relationship does not hold.

Now, embodiments of the present invention will be described. First, a method for evaluating coal of the present embodiment will be described.

<Method for Evaluating Coal>

In the method for evaluating coal of the present embodiment, coal samples having different inert amounts are prepared by pulverizing one brand of coal, and the inert amounts of the respective coal samples are measured. Furthermore, surface tensions of semi-coke, which is obtained by heat treating the respective prepared coal samples, are measured. A ratio of a variation in the surface tensions to a variation in the inert amounts is determined from a regression line that is based on the inert amounts and the surface tensions. By using the ratio as an index, the coal, which is to serve as a raw material for metallurgical coke, is evaluated.

[Estimation of Surface Tension of Semi-coke Prepared from Inert, in which Proportion of Inert Component is 100%, and Surface Tension of Semi-coke Prepared from Reactive, in which Proportion of Inert Component is 0%]

An assumption is made that each of the brands of coal that constitute a coal blend is formed of inert, in which a proportion of an inert component is 100%, and reactive, in which a proportion of the inert component is 0%. Coal samples having different inert amounts are prepared by pulverizing one brand of coal. The inert amounts of the coal samples are measured, and surface tensions of semi-coke obtained by heat treating the respective coal samples are measured. A regression line that is based on the inert amounts and the surface tensions is determined. From the regression line, a surface tension $\gamma_{100}$ and a surface tension $\gamma_0$ are determined. The surface tension $\gamma_{100}$ is a surface tension of the inert, in which a proportion of the inert component is 100%, and the surface tension $\gamma_0$ is a surface tension of the reactive, in which a proportion of the inert component is 0%. $\gamma_{100}$, which is determined in this manner, can be regarded as corresponding to a surface tension of the inert of the coal converted to semi-coke; however, in the present embodiment, $\gamma_{100}$ is referred to as a "surface tension $\gamma_{100}$ of the inert". Similarly, $\gamma_0$ can be regarded as corresponding to a surface tension of the reactive of the coal converted to semi-coke; however, in the present embodiment, $\gamma_0$ is referred to as a "surface tension of the reactive".
Now, a method for determining the regression line will be described. First, the coal samples prepared by pulverizing one brand of coal are sieved. When coal is pulverized, large amounts of inert are in coarse-side particles. Accordingly, the mass fraction of the inert in pulverized coal is varied as a result of the pulverization and sieving. Experiment 1, described below, was conducted to demonstrate that the mass fraction of the inert can be varied, and the regression line can be determined. In Experiment 1, coals A to C and R, which were prepared in the comparative experiment, were used.

<Experiment 1>

Coals A to C and R were pulverized and sieved. Accordingly, coal samples including large amounts of reactive, which is the component other than the inert, and coal samples including large amounts of inert were prepared. These coal samples were subjected to a constituent fraction measurement, which was performed by using a point count method, with an optical microscope, in accordance with JIS M 8816. Accordingly, the total inert TI was measured. Furthermore, semi-coke was prepared by heat treating the respective coal samples at 500°C, and the surface tensions $\gamma$ of the respective types of semi-coke was measured by using the Film Flotation method. The surface tensions $\gamma$ of the semi-coke are a mean value in a surface tension distribution determined by the Film Flotation method. In addition, standard coal samples of coals A to C and R were prepared, which were provided without performing pulverization or sieving, the total inert TI of each of the standard coal samples was measured, and the surface tensions of semi-coke, which was obtained by heat treating the respective samples at 500°C, were measured.
Fig. 2 is a graph illustrating a plot, which shows a relationship between the inert amounts TI of the coal samples and the surface tensions $\gamma$ of semi-coke obtained by heat treating the coal samples, and illustrating regression lines of the plot. Each of the regression lines shown in Fig. 2 is a simple regression line calculated by using a least squares method so that an error with respect to the three plots showing a relationship between the total inert TI and the surface tension $\gamma$ can be minimized. The surface tension $\gamma_0$, which corresponds to an inert amount TI of 0%, and the surface tension $\gamma_{100}$, which corresponds to an inert amount TI of 100%, can be calculated by using the regression line. As an example, the surface tension $\gamma_0$ and surface tension $\gamma_{100}$ of coal samples of coal A are shown in Fig. 2. From Fig. 2, it can be seen that the mass fraction of the inert can be varied by pulverization and sieving, and a linear regression equation with a high correlation between the surface tension $\gamma$ and the inert amount TI can be obtained.
The total inert TI (%) of each of the standard coal samples of coals A to C and R is a volume fraction of the inert of the coal; however, since the specific gravity of the inert is substantially equal to that of the reactive, the volume fraction was dealt with as an approximate equivalent to a mass fraction. The mass fractions (%) of the reactive of the standard coal samples were calculated by subtracting the value of the inert amount TI from the mass fraction (100%) of the entirety. The mass fractions (%) of the inert and the reactive of coals A to C and R and the surface tension $\gamma_{100}$ and the surface tension $\gamma_0$ thereof are shown in Table 3. As described, in the present embodiment, the mass fraction of the inert can be the inert amount (volume fraction) determined by the method of JIS M 8816 and equation [4], and the mass fraction of the reactive can be the value obtained by subtracting the inert amount (volume fraction) from 1.

[Table 3]

| Coal | Constituent | Mass fraction (%) | Surface tension $\gamma_{100}$ (mN/m) | Surface tension $\gamma_0$ (mN/m) |
|---|---|---|---|---|
| A | Inert | 23.6 | 37.0 | - |
| | Reactive | 76.4 | - | 41.3 |
| B | Inert | 18.7 | 41.2 | - |
| | Reactive | 81.3 | - | 39.8 |
| C | Inert | 21.0 | 35.5 | - |
| | Reactive | 79.0 | - | 40.0 |
| R | Inert | 20.4 | 40.8 | - |
| | Reactive | 79.6 | - | 40.3 |

[Evaluation of Coal that Uses, as Index, Variation in Surface Tensions Relative to Variation in Inert Amounts]

The present inventors discovered a phenomenon in which, in cases where the inert amount of coal was varied, a variation in the surface tensions of semi-coke, which was obtained by heat treating the coal, relative to the variation in the inert amounts varied between different brands of coal. For example, in Fig. 2, regarding coal A and coal C, the regression lines thereof had a large slope in absolute value, which indicates that the variation in the surface tensions of semi-coke relative to the variation in the inert amounts was large. Regarding coal B and coal R, the regression lines thereof had a small slope in absolute value, which indicates that the variation in the surface tensions of semi-coke relative to the variation in the inert amounts was small.

Specifically, in an instance where the inert amount is changed from 0% to 100%, the variation in the surface tensions is equal to the difference between $\gamma_{100}$ and $\gamma_0$, that is, $\gamma_{100}$ - $\gamma_0$. $\gamma_{100}$ - $\gamma_0$ of coal A is -4.3 mN/m. $\gamma_{100}$ - $\gamma_0$ of coal C is -4.5 mN/m. On the other hand, $\gamma_{100}$ - $\gamma_0$ of coal B is 1.4 mN/m, and $\gamma_{100}$ - $\gamma_0$ of coal R is 0.5 mN/m. Since the variation in the surface tensions is expressed as the absolute value of $\gamma_{100}$ - $\gamma_0$, coal A and coal C are coals having a high ratio of the variation in the surface tensions of semi-coke to the variation in the inert amounts, compared with coal B and coal R.

As shown in Table 2, in the experimental examples of coal blends 1 to 3, coal R was included in an amount of 50 mass%, and coals A to C were added thereto, respectively, in an amount of 50 mass%. Note that coal blend 2 has the highest coke strength. Coal B, which was added to coal blend 2, is coal having a small variation in the surface tensions of semi-coke relative to the variation in the inert amounts. In contrast, coal blend 1 and coal blend 3, which have a low coke strength, are coal blends in which coal A or coal C, which has a large variation in the surface tensions of semi-coke relative to the variation in the inert amounts, was blended. That is, in the case where coal having a large variation in the surface tensions of semi-coke relative to the variation in the inert amounts was added, the result was that the strength of the coke decreased.

A comparison is made between coal blend 1 and coal blend 4. In coal blend 4, the amount of coal A was reduced by 20% from that in coal blend 1, and coal B was correspondingly added. That is, coal B, which has a small variation in the surface tensions of semi-coke relative to the variation in the inert amounts, replaced a portion corresponding to the 20 mass% of coal A, which has a large variation in the surface tensions of semi-coke relative to the variation in the inert amounts. Since the coke strength of coal blend 4 is higher than the coke strength of coal blend 1, it is apparent that coal having a small variation in the surface tensions of semi-coke relative to the variation in the inert amounts is coal favorable as a raw material for coke that enhances the coke strength.

The results described above indicate that in cases where the variation in the surface tensions of semi-coke relative to the variation in the inert amounts is used as an index, a determination can be made that coal having a small variation in the surface tensions of semi-coke is suitable as a raw material for coke. It is believed that the results are based on an influence of the interfacial tension on the coke strength. That is, coal having a large variation in the surface tensions of semi-coke relative to the variation in the inert amounts has a large difference between the value of $\gamma_{100}$ and the value of $\gamma_0$. The difference means that, assuming that the coal is formed of a component that softens and melts and a component that does not soften or melt, the surface tensions of semi-coke derived from the respective two components are significantly different. When it is assumed that one brand of coal is formed of a component that softens and melts and a component that does not soften or melt rather than being homogeneous, in a case where a raw material for coke is formed by blending the coal, the raw material includes components having significantly different surface tensions of semi-coke. As the difference in the surface tension increases, the interfacial tension increases, and, therefore, the interfacial tension $\gamma_{blend}$ of a coal blend including components having significantly different surface tensions of semi-coke is high, which reduces adhesiveness at interfaces. As a result, presumably, the coke strength is adversely affected.

Now, embodiments of a method for preparing a coal blend will be described. There are three embodiments of the method for preparing a coal blend. In the method for preparing a coal blend of a first embodiment, an interfacial tension is calculated, and a correlation between the interfacial tension and the strength of coke is determined; from the correlation, an interfacial tension corresponding to a desired strength of the coke is determined; and a coal blend is prepared by mixing coal in mass fractions such that the interfacial tension or a lower interfacial tension is achieved. In the method for preparing a coal blend of a second embodiment, a coal blend is prepared by mixing coal in mass fractions such that an interfacial tension of 0.26 mN/m or less as calculated is achieved. In the method for preparing a coal blend of a third embodiment, a coal blend is prepared in a manner such that a blending ratio of unfavorable coal as evaluated by the above-described method for evaluating coal is limited. First, the method for preparing a coal blend of the first embodiment will be described.

<Method for Preparing Coal Blend of First Embodiment>

The method for preparing a coal blend of the first embodiment includes steps 1 to 3 as described below.

[Step 1]

In step 1, an assumption is made that each of the brands of coal that constitute a coal blend is formed of inert, in which a proportion of an inert component is 100%, and reactive, in which a proportion of the inert component is 0%. Coal samples having different inert amounts are prepared by pulverizing one brand of coal, and the inert amounts of the respective coal samples are measured. In addition, surface tensions of semi-coke obtained by heat treating the respective coal samples are measured, a regression line that is based on the inert amounts and the surface tensions is determined, and, from the regression line, a surface tension $\gamma_{100}$, which corresponds to an inert component content of 100%, and a surface tension $\gamma_0$, which corresponds to an inert component content of 0%, are determined.

[Step 2]

In step 2, following step 1, an assumption is made that a surface tension of the semi-coke derived from the inert is equal to the surface tension $\gamma_{100}$, and surface tensions of semi-coke derived from the melt and the reactive are equal to the surface tension $\gamma_0$, and an interfacial tension $\gamma_{blend}$ is calculated from the mass fraction of the inert, the mass fraction of the reactive, the surface tension $\gamma_{100}$, and the surface tension $\gamma_0$ of each of the brands by using equation [1] or equation [2], and equation [3].

In the present embodiment, an assumption is made that one brand of coal is formed of two types of coal, namely, coal formed of inert, in which a proportion of the inert component is 100%, and coal formed of reactive, in which a proportion of the inert component is 0%, and an assumption is made that a surface tension of semi-coke derived from the coal formed of inert is equal to the surface tension $\gamma_{100}$, and a surface tension of semi-coke derived from the coal formed of reactive is equal to the surface tension $\gamma_0$. In the present embodiment, based on these assumptions, the interfacial tension $\gamma_{blend}$ is calculated by using equation [1] or [2] and equation [3].

That is, in the present embodiment, in an instance where n brands of coal are actually present in a coal blend, the following assumptions are made: 2 (types) × n (number of brands) types of coal are present, with the two being coal formed of inert and coal formed of reactive; and the mass fractions of the coal formed of inert and the coal formed of reactive are denoted as $w_i$ (which represents the mass fractions of 1st, 2nd,...ith,...and 2×nth coal). By substituting the surface tension $\gamma_{100}$ and the surface tension $\gamma_0$ of the types of coal, which are assumed to be present, for $\gamma_i$ and $\gamma_j$ in equation [1] or [2], an interfacial tension $\gamma_{ij}$, which is an interfacial tension between two types of semi-coke derived from two brands of coal, can be calculated. The interfacial tension $\gamma_{blend}$ can be calculated from the interfacial tension $\gamma_{ij}$ by using equation [3]. Regarding equation [3], the mass fractions $w_i$ and $w_j$ of the coal formed of inert and the coal formed of reactive in a coal blend can be calculated by multiplying the mass fraction of the one brand of coal of the coal blend by each of the mass fractions of the inert and the reactive of a standard coal sample of the coal (see Table 3, for example).

Experiment 2 was conducted as follows. Regarding coal blends 5 to 14, each of which is formed of two or more of coals D to N, listed in Table 4, assuming that the two types of coal, namely, coal formed of inert and coal formed of reactive, are present as described above, the interfacial tension $\gamma_{blend}$ is calculated, and a relationship between the interfacial tension $\gamma_{blend}$ and the coke strength DI150/15 (-) is determined. The analysis values shown in Table 4 are those measured by the same method as that described in the description of Table 1. The surface tension is a mean value in a surface tension distribution obtained by measuring, by using the Film Flotation method, surface tensions of semi-coke, which resulted from heat treatment of the respective types of coal at 500°C. The surface tension of the inert, in which a proportion of the inert component is 100%, and the surface tension of the reactive, in which a proportion of the inert component is 0%, are the values of $\gamma_{100}$ and $\gamma_0$, respectively, as calculated by the method described in experiment 1.

[Table 4]

| Brand name | log MF (log/ddpm) | Ro (%) | TI (%) | Volatile matter (wt% d.b) | Ash (wt% d.b) | Surface tension (mN/m) | Surface tension of inert (mN/m) | Surface tension of reactive (mN/m) |
|---|---|---|---|---|---|---|---|---|
| D | 2.95 | 1.10 | 37.8 | 25.7 | 9.3 | 40.1 | 43.1 | 38.3 |
| E | 2.48 | 1.24 | 38.4 | 23.3 | 8.4 | 39.3 | 41.2 | 38.1 |
| F | 1.04 | 1.23 | 45.6 | 21.6 | 8.0 | 40.2 | 41.2 | 39.3 |
| G | 2.77 | 0.97 | 43.4 | 27.0 | 9.2 | 40.9 | 44.9 | 37.9 |
| H | 2.58 | 0.98 | 33.8 | 27.1 | 8.3 | 41.1 | 44.8 | 39.2 |
| I | 0.48 | 0.99 | 46.6 | 25.7 | 9.8 | 41.3 | 44.7 | 38.4 |
| J | 1.79 | 0.97 | 32.4 | 29.0 | 8.7 | 40.2 | 44.9 | 37.9 |
| K | 0.85 | 1.54 | 21.4 | 18.6 | 10.7 | 38.7 | 37.1 | 39.1 |
| L | 3.47 | 0.64 | 21.8 | 42.1 | 6.8 | 41.6 | 49.4 | 39.4 |
| M | 2.85 | 1.18 | 30.8 | 23.3 | 10.4 | 39.8 | 42.0 | 38.8 |
| N | 2.65 | 1.17 | 29.2 | 22.8 | 10.2 | 39.8 | 42.1 | 38.8 |

<Experiment 2>

The blending ratio of each of the brands, the grades of the coal blends, and the strength DI(150/15) of coke obtained by carbonizing the respective coal blends are shown in Table 5. The values of the log MF, Ro, TI, the ash, and the volatile matter are weighted averages, according to the blending ratio, of the analysis values of the types of coal included in the coal blend. "$\gamma_{blend}$" is a value calculated by the same method as the method described in [Step 2]. In the present example, low-value coal was defined as coal having an absolute value of $\gamma_{100} - \gamma_0$ of 6 mN/m or greater; $\gamma_{100} - \gamma_0$ represents a variation in the surface tensions of semi-coke relative to a variation in the inert amounts. Accordingly, a low-value coal ratio is the sum of the blending ratios of coal having an absolute value of $\gamma_{100} - \gamma_0$ of 6 mN/m or greater.

In the blending examples of Table 5, coal was blended such that Ro of the coal blend was approximately 1.03%. In the blending examples of Table 2, coal having Ro of 1.20 to 1.59% was used, and Ro of the coal blends were 1.30 to 1.40%. However, regarding a coal blend for the production of coke, it is common to blend together a large number of brands of coal (5 brands to 20 brands) having different coal properties, and limiting the coal to be blended to a small number of brands is not preferable because in such a case, the flexibility of operation is restricted as a result of the restriction on the coal to be blended. Since coal having a high Ro tends to be expensive, in experiment 2, coal blends having a lower Ro than the Ro's of the blending examples of Table 2 were used because experiment 2 was designed to use actual operational conditions.

In the present embodiment, the grades of the coal blends are not limited to the examples shown in Table 5. Regarding an average grade of the coal blend, the following ranges may be employed so that the present invention can be suitably used: Ro, 0.9 to 1.4%; log MF, 1.7 to 3.0 (logddpm); and TI, 15 to 40%. Particularly preferably, the ranges are as follows: Ro, 0.9 to 1.3%; log MF, 2.0 to 3.0 (logddpm); and TI, 20 to 40%. Regarding the grades of the individual brands of coal, the coal may be as follows so that the present invention can be suitably used: Ro, 0.6 to 1.7 (%); MF, 0 to 60000 ddpm; TI, 3 to 45 (%); volatile matter, 3 to 45%; ash, 1 to 20%; and a surface tension (a mean value in a distribution), 36 to 46 mN/m.

[Table 5]

| Coal | Coal blend 5 | Coal blend 6 | Coal blend 7 | Coal blend 8 | Coal blend 9 | Coal blend 10 | Coal blend 11 | Coal blend 12 | Coal blend 13 | Coal blend 14 |
|---|---|---|---|---|---|---|---|---|---|---|
| D | 30.0 | | | | 30.0 | | 30.0 | | | |
| E | | 30.0 | | | | 30.0 | | 30.0 | | |
| F | | | 30.0 | | | | | | 30.0 | |
| G | | | | 30.0 | | | | | | 30.0 |
| H | | | 7.0 | | | | | | 25.0 | |

(continued)

| Coal | Coal blend 5 | Coal blend 6 | Coal blend 7 | Coal blend 8 | Coal blend 9 | Coal blend 10 | Coal blend 11 | Coal blend 12 | Coal blend 13 | Coal blend 14 |
|---|---|---|---|---|---|---|---|---|---|---|
| I | 18.6 | 18.0 | 8.0 | 16.0 | 15.8 | 17.6 | 13.0 | 17.3 | 0.0 | 11.0 |
| J | 25.1 | 25.0 | 10.5 | 23.0 | 24.1 | 16.0 | 23.0 | 7.9 | 0.0 | 17.0 |
| K | 4.9 | 1.1 | 1.0 | 4.7 | 2.5 | 0.7 | 0.0 | 0.0 | 0.0 | 0.0 |
| L | 9.6 | 14.9 | 21.6 | 4.0 | 9.4 | 18.5 | 9.2 | 21.4 | 21.4 | 5.0 |
| M | 0.0 | 0.0 | 18.4 | 8.0 | 8.4 | 10.4 | 16.8 | 20.4 | 23.6 | 19.0 |
| N | 11.8 | 11.0 | 3.5 | 14.3 | 9.9 | 6.8 | 8.0 | 3.0 | 0.0 | 18.0 |
| log MF (log/ddpm) | 2.11 | 2.10 | 2.09 | 2.11 | 2.24 | 2.24 | 2.37 | 2.37 | 2.37 | 2.38 |
| Ro (%) | 1.033 | 1.034 | 1.030 | 1.032 | 1.033 | 1.033 | 1.033 | 1.033 | 1.029 | 1.032 |
| TI (%) | 34.7 | 34.7 | 34.8 | 36.4 | 34.5 | 34.3 | 34.4 | 34.0 | 34.1 | 35.9 |
| Ash (wt% d.b) | 9.2 | 8.7 | 8.5 | 9.4 | 9.2 | 8.7 | 9.3 | 8.8 | 8.4 | 9.5 |
| Volatile matter (wt% d.b) | 27.4 | 27.9 | 27.8 | 26.6 | 27.4 | 28 | 27.3 | 28.2 | 27.8 | 26.5 |
| $\gamma_{blend}$ | 0.28 | 0.29 | 0.24 | 0.31 | 0.27 | 0.28 | 0.26 | 0.27 | 0.22 | 0.28 |
| Low-value coal ratio (%) | 53.3 | 57.9 | 40.1 | 73.0 | 49.3 | 52.1 | 45.2 | 46.6 | 21.4 | 63.0 |
| DI150/15 (-) | 78.7 | 79.4 | 80.3 | 78.6 | 79.1 | 79.7 | 80.5 | 80.0 | 80.6 | 79.3 |

Regarding Table 5, $\gamma_{blend}$ was calculated as follows. For example, in the case of coal D in coal blend 5 of Table 5, the blending ratio of coal D was 30%. For coal blend 5, an assumption was made that coal D was formed of two types of coal, namely, coal formed of inert and coal formed of reactive. The blending ratio of the coal formed of inert of coal D was determined to be 11.3%; this was derived by multiplying the blending ratio of coal D, which was 30%, by the ratio of the inert in coal D, which was 37.8%. The blending ratio of the coal formed of reactive of coal D was determined to be 18.7%; this was derived by multiplying the blending ratio of coal D, which was 30%, by the ratio of the reactive in coal D, which was 62.2%. In this manner, for each of the types of coal, the coal formed of inert and the coal formed of reactive were each dealt with as if the coal was a single brand of coal, and $\gamma_{blend}$ was calculated by using equation [3].

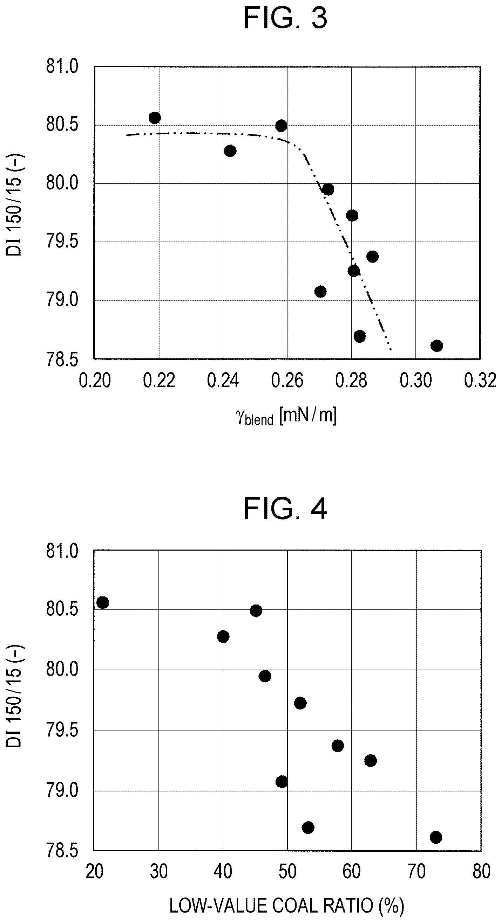

Fig. 3 is a graph illustrating a relationship between an interfacial tension $\gamma_{blend}$, which was calculated by the method of the present invention, and the coke strength DI150/15 (-). As indicated by the dash-dot-dot curve of Fig. 3, it is apparent that a correlation exists between the interfacial tension $\gamma_{blend}$ and the coke strength DI150/15 (-); that is, as the interfacial tension $\gamma_{blend}$ increases, the coke strength DI150/15 (-) decreases. The curve can be derived by drawing a correlation curve by using a least squares method or freehand in a graph such as that of Fig. 3.

[Step 3]

In step 3, an interfacial tension $\gamma_{blend}$ corresponding to a desired strength is determined from the correlation determined in step 2. Since as an interfacial tension $\gamma_{blend}$ increases, adhesion between coal particles decreases, a coal blend is to be prepared by mixing together the brands of coal in mass fractions such that the determined interfacial tension $\gamma_{blend}$ or a lower interfacial tension is achieved. In an instance where the interfacial tension is calculated from the surface tensions of semi-coke derived from coal that constitutes a coal blend and the mass fractions of the coal, and the calculated interfacial tension is equal to or less than the interfacial tension corresponding to a desired coke strength, a coal blend is to be prepared by mixing together plural brands of coal in the mass fractions. Coke produced by carbonizing a coal blend prepared in this manner can be expected to have a desired or greater strength.

The coal to be used in the preparation of a coal blend in step 3 may be different from the coal used in experiment 2 regarding step 2. Step 2 is implemented to determine a correlation between $\gamma_{blend}$ of a coal blend and the coke strength by conducting, preliminarily, a coke production test that uses a particular coal blend. In step 3, based on the correlation predetermined in step 2, coal can be freely selected such that $\gamma_{blend}$ equal to or less than a value at which a desired strength is provided is achieved. In this instance, it is preferable to ensure that the average properties of the coal blend used in the coke production test, which is conducted preliminarily in step 2, have values close to those of the average properties of the coal blend that is prepared in step 3. In this case, the strength of coke derived from the coal blend prepared in step 3 can be predicted more accurately. For example, it is more preferable to ensure that the differences between the average properties of the coal blend used in step 2 and the average properties of the coal blend prepared in step 3 are as follows: difference in the mean reflectance, less than or equal to 0.2%; and difference in log MF, less than or equal to 1.0 (logddpm). It is more preferable that the test of step 2 be conducted by using one-half or more of the number of types of coal used in step 3. However, regardless of the properties of a coal blend, as long as $\gamma_{blend}$ of the coal blend is equal to or less than the value of $\gamma_{blend}$ at which a desired coke strength is provided, production of coke having higher strength than in the case of a coal blend having a $\gamma_{blend}$ value higher than or equal to the mentioned $\gamma_{blend}$ value can be expected.

In the examples shown in Table 5 and Fig. 3, coke with a preferred coke strength of 78.5 to 80.5 can be produced. If necessary, higher coke strength can be achieved by changing the type of coal to be used and/or preparing the grade of the coal blend. Specifically, since the coke strength increases when Ro of the coal blend is increased, a coke production test that employs a higher Ro of the coal blend than the Ro's of the examples of Table 5 to change the value of $\gamma_{blend}$ may be conducted, and under the blending conditions, the relationship between $\gamma_{blend}$ and the coke strength may be determined; based on the relationship, $\gamma_{blend}$ for producing coke having a desired strength can be determined.

In the method for preparing a coal blend of the first embodiment, an agent that performs steps 1 and 2 may be different from an agent that performs step 3. Step 1 and step 2 may be carried out in advance, and, in step 3, the interfacial tension $\gamma_{blend}$ may be determined based on the predetermined correlation. That is, even when the agent that performs step 1 and step 2 is different from the agent that performs step 3, the method for preparing a coal blend of the first embodiment can be implemented. Likewise, an agent that performs step 1 may be different from an agent that performs step 2 and step 3. That is, the correlation between the coke strength and $\gamma_{blend}$ may be determined based on predetermined surface tensions $\gamma_{100}$ and $\gamma_0$. Now, the method for preparing a coal blend of the second embodiment will be described.

<Method for Preparing Coal Blend of Second Embodiment>

The method for preparing a coal blend of the second embodiment includes steps $\alpha$ and $\beta$ as described below.

[Step $\alpha$]

In step $\alpha$, an assumption is made that each of the brands of coal that constitute a coal blend is formed of inert, in which a proportion of the inert component is 100%, and reactive, in which a proportion of the inert component is 0%. Thereafter, coal samples having different inert amounts are prepared by pulverizing one brand of coal, and the inert amounts of the respective coal samples are measured. In addition, surface tensions of semi-coke obtained by heat treating the respective coal samples are measured, a regression line that is based on the inert amounts and the surface tensions is determined, and, from the regression line, a surface tension $\gamma_{100}$, which corresponds to an inert component content of 100%, and a surface tension $\gamma_0$, which corresponds to an inert component content of 0%, are determined. Step $\alpha$ is the same as [Step 1] for the method for preparing a coal blend of the first embodiment, and, therefore, redundant descriptions are omitted.

[Step $\beta$]

In step $\beta$, an assumption is made that a surface tension of semi-coke derived from the inert is equal to the surface tension $\gamma_{100}$, and a surface tension of semi-coke derived from the reactive is equal to the surface tension $\gamma_0$. Thereafter, an interfacial tension $\gamma_{blend}$ is calculated from the blending ratio of each of the brands, the mass fractions of the inert and the reactive, the surface tension $\gamma_{100}$, and the surface tension $\gamma_0$ by using equation [3]. This step, which is for determining the interfacial tension, is the same as [Step 2] of the method for preparing a coal blend of the first embodiment, and, therefore, redundant descriptions are omitted.

In step $\beta$, a mass fraction of each of the brands of coal that constitute a coal blend is specified such that an interfacial tension of 0.26 mN/m or less as calculated is achieved. As indicated by the dash-dot-dot curve of Fig. 3, in cases where a coal blend is prepared by blending together plural brands of coal in specified mass fractions such that the calculated interfacial tension $\gamma_{blend}$ is 0.26 mN/m or less, a reduction in the strength of coke produced by carbonizing the coal blend can be inhibited, and, consequently, production of coke having high strength can be expected. The results of Fig. 3 are based on the results of preparation of semi-coke carried out by heat treating coal at 500°C. Accordingly, it is preferable that the value of the surface tension of semi-coke prepared at 500°C be used. However, the value of the interfacial tension does not significantly change with a change in the semi-coke preparation temperature. Accordingly, even in instances where semi-coke is prepared at a different temperature, production of coke having high strength can be realized by ensuring that the coal blend has an interfacial tension value of 0.26 mN/m or less.

In the method for preparing a coal blend of the second embodiment, step $\alpha$ may be implemented in advance to determine the surface tension $\gamma_{100}$ and the surface tension $\gamma_0$ of the coal, and step $\beta$ may be implemented by using the predetermined surface tension $\gamma_{100}$ and surface tension $\gamma_0$. That is, even when an agent that performs step $\alpha$ is different from an agent that performs step $\beta$, the method for preparing a coal blend of the second embodiment can be implemented. Now, the method for preparing a coal blend of the third embodiment will be described.

<Method for Preparing Coal Blend of Third Embodiment>

The method for preparing a coal blend of the third embodiment includes steps A and B as described below. In the third embodiment, a variation in the surface tensions relative to a variation in the inert amounts is used as an index. As shown in Table 2, coal having a small variation in the surface tensions of semi-coke relative to the variation in the inert amounts is evaluated as being suitable as a raw material for coke, and coal having a large variation in the surface tensions of semi-coke relative to the variation in the inert amounts is evaluated as not being suitable as a raw material for coke. In Table 2, the number of types of coal used is small and limited, and the values of Ro of the coal blends are greater than the values of commonly used coal blends. Accordingly, based on the results of Table 5, which were obtained under the blending conditions closer to actual blending conditions, studies were conducted for criteria for the variation in the surface tensions of semi-coke relative to the variation in the inert amounts, which is used to evaluate coal.

[Step A]

In step A of the third embodiment, an assumption is made that each of the brands of coal that constitute a coal blend is formed of inert, in which a proportion of the inert component is 100%, and reactive, in which a proportion of the inert component is 0%. Thereafter, coal samples having different inert amounts are prepared by pulverizing one brand of coal, and the inert amounts of the respective coal samples are measured. In addition, surface tensions of semi-coke obtained by heat treating the respective coal samples are measured, a regression line that is based on the inert amounts and the surface tensions is determined, and, from the regression line, a surface tension $\gamma_{100}$, which corresponds to an inert component content of 100%, and a surface tension $\gamma_0$, which corresponds to an inert component content of 0%, are determined. Step A is the same as [Step 1] of the method for preparing a coal blend of the first embodiment and [Step $\alpha$] of the method for preparing a coal blend of the second embodiment, and, therefore, redundant descriptions are omitted.

[Step B]

In step B, an assumption is made that a surface tension of semi-coke derived from the inert is equal to the surface tension $\gamma_{100}$, and a surface tension of semi-coke derived from the reactive is equal to the surface tension $\gamma_0$. Thereafter, coal unfavorable as a raw material for coke is determined based on the absolute value of the difference between $\gamma_{100}$ and $\gamma_0$, and a coal blend is prepared such that the blending ratio of the unfavorable coal is low. Of coals D to N listed in Table 4, coal having a difference between $\gamma_{100}$ and $\gamma_0$ of 6 mN/m or greater was rated as low-value coal, which is unsuitable as a raw material for coke, and coal having a difference between $\gamma_{100}$ and $\gamma_0$ of less than 6 mN/m was rated as high-value coal, which is suitable as a raw material for coke. A relationship between the blending ratio of low-value coal and the coke strength was examined.

Fig. 4 is a graph illustrating a relationship between the coke strength and the blending ratio of low-value coal. As shown in Fig. 4, it is apparent that a high correlation exists between the coke strength and the blending ratio of low-value coal having a difference between $\gamma_{100}$ and $\gamma_0$ of 6 mN/m or greater. That is, coal having an absolute value of $\gamma_{100} - \gamma_0$ of 6 mN/m or greater is evaluated as low-value coal unfavorable as a raw material for coke. Accordingly, a coal blend is to be prepared by mixing coal in a manner such that the blending ratio of the low-value coal is less than or equal to 45 mass%. In cases where a coal blend is prepared in this manner, the resulting coal blend is a coal blend from which coke having high strength is expected to be produced. This result is also based on the value of a surface tension of semi-coke prepared at 500°C. However, the value of semi-coke prepared at a different temperature may be used, and in such a case, too, a similar evaluation can be made. Coal having an absolute value of $\gamma_{100} - \gamma_0$ of 6 mN/m or greater can be evaluated as low-value coal unfavorable as a raw material for coke, and, therefore, it is preferable that the blending ratio of such coal be as low as possible. That is, it is preferable that the lower limit of the blending ratio of coal having an absolute value of $\gamma_{100} - \gamma_0$ of 6 mN/m or greater be 0%.

In the method for preparing a coal blend of the third embodiment, step A may be implemented in advance to determine the surface tension $\gamma_{100}$ and the surface tension $\gamma_0$ of the coal, and step B may be implemented by using the predetermined surface tension $\gamma_{100}$ and surface tension $\gamma_0$. That is, even when an agent that performs step A is different from an agent that performs step B, the method for preparing a coal blend of the third embodiment can be implemented.

As described, with the present invention, whether a brand of coal is coal favorable as a raw material for metallurgical coke can be evaluated. In addition, types of coal that are to constitute a coal blend from which coke having a desired strength can be produced and mass fractions of the types of coal can be accurately specified compared with related-art techniques. As a result, the production of coke having a desired strength can be realized.

**Claims**

1. A method for evaluating coal, the method comprising:

preparing coal samples having different inert amounts by pulverizing one brand of coal;
measuring the inert amounts of the respective coal samples and measuring surface tensions of semi-coke obtained by heat treating the respective coal samples; and
determining a regression line that is based on the inert amounts and the surface tensions and evaluating the coal by using, as an index, a ratio of a variation in the surface tensions to a variation in the inert amounts as determined from the regression line, wherein the inert amounts are calculated by using the method for measuring macerals of coal according to JIS M 8816 and equation [4]: inert amount (vol%) = fusinite (vol%) + micrinite (vol%) + $(2/3) \times$ semifusinite (vol%) + mineral matter (vol%) as described in the description, the surface tension is measured according to the Film Flotation method as described in the description, and wherein semi-coke is a heat-treated product obtained by heat treating coal as described in the description.

2. A method for preparing a coal blend, the method being a method for preparing a coal blend that includes at least two brands of coal, the method comprising:

determining a surface tension $\gamma_{100}$ and a surface tension $\gamma_0$ of each of brands of coal of a coal blend from a regression line determined by the method for evaluating coal according to Claim 1, the surface tension $\gamma_{100}$ corresponding to an inert amount of 100%, the surface tension $\gamma_0$ corresponding to an inert amount of 0%;
determining an interfacial tension of the coal blend from the surface tension $\gamma_{100}$, the surface tension $\gamma_0$, a blending ratio of each of the brands of coal of the coal blend, and a mass fraction of inert and a mass fraction of reactive in each of the brands of coal, wherein the surface tension $\gamma_{100}$, the surface tension $\gamma_0$, a blending ratio of each of the brands of coal of the coal blend, and a mass fraction of inert and a mass fraction of reactive in each of the brands of coal are determined utilizing the equations and methods described in the description;
determining a correlation between the interfacial tension and a strength of coke produced from the coal blend;
determining, from the correlation, an interfacial tension corresponding to a desired strength of the coke;
determining mass fractions of the at least two brands of coal such that the interfacial tension corresponding to a desired strength of the coke or a lower interfacial tension is achieved; and
preparing a coal blend by mixing together the at least two brands of coal in the mass fractions.

3. A method for preparing a coal blend, the method being a method for preparing a coal blend that includes at least two brands of coal, the method comprising:

determining a surface tension $\gamma_{100}$ and a surface tension $\gamma_0$ of each of brands of coal of a coal blend from a regression line determined by the method for evaluating coal according to Claim 1, the surface tension $\gamma_{100}$ corresponding to an inert amount of 100%, the surface tension $\gamma_0$ corresponding to an inert amount of 0%;
determining mass fractions of the at least two brands of coal such that an interfacial tension of 0.26 mN/m or less, as calculated from the surface tension $\gamma_{100}$, the surface tension $\gamma_0$, a blending ratio of each of the brands of coal of the coal blend, and a mass fraction of inert and a mass fraction of reactive in each of the brands of coal, is achieved, wherein the surface tension $\gamma_{100}$, the surface tension $\gamma_0$, a blending ratio of each of the brands of coal of the coal blend, and a mass fraction of inert and a mass fraction of reactive in each of the brands of coal are determined utilizing the equations and methods described in the description; and
preparing a coal blend by mixing together the at least two brands of coal in the mass fractions.

4. A method for preparing a coal blend, the method being a method for preparing a coal blend that includes at least two brands of coal, the method comprising:

determining a surface tension $\gamma_{100}$ and a surface tension $\gamma_0$ of each of brands of coal of a coal blend from a regression line determined by the method for evaluating coal according to Claim 1, the surface tension $\gamma_{100}$ corresponding to an inert amount of 100%, the surface tension $\gamma_0$ corresponding to an inert amount of 0%, wherein the surface tension $\gamma_{100}$ and the surface tension $\gamma_0$ are determined utilizing the equations and methods described in the description; and
preparing a coal blend by mixing together the at least two brands of coal in a manner such that a mass fraction of coal having an absolute value of a difference between the surface tension $\gamma_{100}$ and the surface tension $\gamma_0$ of 6 mN/m or greater is less than or equal to 45 mass% in the coal blend.

5. A method for producing coke, the method comprising:

preparing a coal blend by using the method for preparing a coal blend according to any one of Claims 2 to 4; and
producing coke by carbonizing the coal blend.

**Patentansprüche**

1. Ein Verfahren zur Bewertung von Kohle, wobei das Verfahren aufweist:

Herstellen von Kohleproben mit unterschiedlichen Inertmengen durch Pulverisieren einer Kohlesorte;
Messen der Inertmengen der jeweiligen Kohleproben und Messen der Oberflächenspannungen des durch Wärmebehandlung der jeweiligen Kohleproben erhaltenen Halbkokses; und
Bestimmen einer Regressionslinie, die auf den Inertmengen und den Oberflächenspannungen basiert, und Bewerten der Kohle unter Verwendung eines Verhältnisses einer Variation in den Oberflächenspannungen zu einer Variation in den Inertmengen, wie sie von der Regressionslinie bestimmt werden, als ein Index, wobei die Inertmengen unter Verwendung des Verfahrens zum Messen von Makeralien von Kohle gemäß JIS M 8816 und Gleichung [4] berechnet werden:
Inertmenge (Vol.-%) = Fusinit (Vol.-%) + Mikrinit (Vol.-%) + (2/3)xSemifusinit (Vol.-%) + Mineralstoffe (Vol.-%), wie in der Beschreibung beschrieben, die Oberflächenspannung wird nach dem Filmflotationsverfahren, wie in der Beschreibung beschrieben, gemessen, und wobei Halbkoks ein wärmebehandeltes Produkt ist, das durch Wärmebehandlung von Kohle, wie in der Beschreibung beschrieben, erhalten wird.

2. Ein Verfahren zur Herstellung einer Kohlemischung, wobei das Verfahren ein Verfahren zur Herstellung einer Kohlemischung ist, die mindestens zwei Kohlensorten aufweist, wobei das Verfahren aufweist:

Bestimmen einer Oberflächenspannung $\gamma_{100}$ und einer Oberflächenspannung $\gamma_0$ jeder der Kohlemarken einer Kohlemischung aus einer Regressionslinie, die durch das Verfahren zur Bewertung von Kohle nach Anspruch 1 bestimmt wird, wobei die Oberflächenspannung $\gamma_{100}$ einer Inertmenge von 100 % entspricht und die Oberflächenspannung $\gamma_0$ einer Inertmenge von 0 % entspricht;
Bestimmen einer Grenzflächenspannung der Kohlemischung aus der Oberflächenspannung $\gamma_{100}$, der Oberflächenspannung $\gamma_0$, einem Mischungsverhältnis jeder der Kohlemarken der Kohlemischung und einem Massenanteil an inertem und einem Massenanteil an reaktivem Material in jeder der Kohlemarken, wobei die Oberflächenspannung $\gamma_{100}$, die Oberflächenspannung $\gamma_0$, ein Mischungsverhältnis jeder der Kohlemarken der Kohlemischung und ein Massenanteil an inertem und ein Massenanteil an reaktivem Material in jeder der Kohlemarken unter Verwendung der in der Beschreibung beschriebenen Gleichungen und Verfahren bestimmt werden;
Bestimmen einer Korrelation zwischen der Grenzflächenspannung und einer Festigkeit des aus der Kohlemischung hergestellten Kokses;
Bestimmen einer Grenzflächenspannung, die einer gewünschten Festigkeit des Kokses entspricht, aus der Korrelation;
Bestimmen von Massenanteilen der mindestens zwei Kohlesorten, so dass die Grenzflächenspannung, die einer gewünschten Festigkeit des Koks entspricht, oder eine geringere Grenzflächenspannung erreicht wird; und
Herstellen einer Kohlemischung durch Zusammenmischen der mindestens zwei Kohlesorten in den Massenanteilen.

3. Ein Verfahren zur Herstellung einer Kohlemischung, wobei das Verfahren ein Verfahren zur Herstellung einer Kohlemischung ist, die mindestens zwei Kohlesorten aufweist, wobei das Verfahren aufweist:

Bestimmen einer Oberflächenspannung $\gamma_{100}$ und einer Oberflächenspannung $\gamma_0$ jeder der Kohlemarken einer Kohlemischung aus einer Regressionslinie, die durch das Verfahren zur Bewertung von Kohle nach Anspruch 1 bestimmt wird, wobei die Oberflächenspannung $\gamma_{100}$ einer Inertmenge von 100 % entspricht, die Oberflächenspannung $\gamma_0$ einer Inertmenge von 0 % entspricht;

Bestimmen von Massenanteilen der mindestens zwei Kohlesorten, so dass eine Grenzflächenspannung von 0.26 mN/m oder weniger, berechnet aus der Oberflächenspannung $\gamma_{100}$, der Oberflächenspannung $\gamma_0$, einem Mischungsverhältnis jeder der Kohlemarken der Kohlemischung und einem Massenanteil an inertem und einem Massenanteil an reaktivem Material in jeder der Kohlemarken, erreicht wird, wobei die Oberflächenspannung $\gamma_{100}$, die Oberflächenspannung $\gamma_0$, ein Mischungsverhältnis jeder der Kohlemarken der Kohlemischung und ein Massenanteil an inertem und ein Massenanteil an reaktivem Material in jeder der Kohlemarken unter Verwendung der in der Beschreibung beschriebenen Gleichungen und Verfahren bestimmt werden; und
Herstellen einer Kohlemischung durch Zusammenmischen der mindestens zwei Kohlesorten in den Massenanteilen.

4. Ein Verfahren zur Herstellung einer Kohlemischung, wobei das Verfahren ein Verfahren zur Herstellung einer Kohlemischung ist, die mindestens zwei Kohlesorten aufweist, wobei das Verfahren aufweist:

Bestimmen einer Oberflächenspannung $\gamma_{100}$ und einer Oberflächenspannung $\gamma_0$ jeder der Kohlemarken einer Kohlemischung aus einer Regressionslinie, die durch das Verfahren zur Bewertung von Kohle gemäß Anspruch 1 bestimmt wird, wobei die Oberflächenspannung $\gamma_{100}$ einer Inertmenge von 100 % entspricht, die Oberflächenspannung $\gamma_0$ einer Inertmenge von 0 % entspricht, wobei die Oberflächenspannung $\gamma_{100}$ und die Oberflächenspannung $\gamma_0$ unter Verwendung der in der Beschreibung beschriebenen Gleichungen und Verfahren bestimmt werden; und
Herstellen einer Kohlemischung durch Zusammenmischen der mindestens zwei Kohlesorten in einer solchen Weise, dass ein Massenanteil von Kohle mit einem Absolutwert einer Differenz zwischen der Oberflächenspannung $\gamma_{100}$ und der Oberflächenspannung $\gamma_0$ von 6 mN/m oder mehr kleiner oder gleich 45 Massenprozent in der Kohlemischung ist.

5. Ein Verfahren zur Herstellung von Koks, wobei das Verfahren aufweist:

Herstellen einer Kohlemischung durch Anwendung des Verfahrens zur Herstellung einer Kohlemischung gemäß einem der Ansprüche 2 bis 4; und
Herstellen von Koks durch Karbonisieren der Kohlemischung.

**Revendications**

1. Procédé d'évaluation de charbon, le procédé comprenant :

une préparation d'échantillons de charbon présentant différentes quantités inertes en pulvérisant une marque de charbon ;
une mesure des quantités inertes des échantillons de charbon respectifs et une mesure de tensions de surface de semi-coke obtenu par un traitement thermique des échantillons de charbon respectifs ; et
une détermination d'une ligne de régression qui repose sur les quantités inertes et les tensions de surface, et une évaluation du charbon en utilisant, comme index, un rapport d'une variation dans les tensions de surface à une variation dans les quantités inertes comme déterminé à partir de la ligne de régression, dans lequel les quantités inertes sont calculées en utilisant la méthode de mesure des macéraux de charbon selon la norme JIS M 8816 et une équation [4] :
quantité inerte (%vol) = fusinite (%vol) + micrinite (%vol) + (2/3) x semifusinite (%vol) + matière minérale (%vol) comme décrit dans la description, la tension de surface est mesurée selon le procédé de flottation de film comme décrit dans la description, et dans lequel le semi-coke est un produit traité thermiquement obtenu par un traitement thermique du charbon comme décrit dans la description.

2. Procédé de préparation d'un mélange de charbon, le procédé consistant en un procédé pour une préparation d'un mélange de charbon qui inclut au moins deux marques de charbon, le procédé comprenant :

une détermination d'une tension de surface $\gamma_{100}$ et d'une tension de surface $\gamma_0$ de chacune des marques de charbon d'un mélange de charbon issues d'une ligne de régression déterminée par le procédé pour une évaluation de charbon selon la revendication 1, la tension de surface $\gamma_{100}$ correspondant à une quantité inerte de 100 %, la surface de tension $\gamma_0$ correspondant à une quantité inerte de 0 % ;
une détermination d'une tension interfaciale du mélange de charbon à partir de la tension de surface $\gamma_{100}$, la tension de surface $\gamma_0$, un rapport de mélange de chacune des marques de charbon du mélange de charbon, et une fraction de masse d'inerte et une fraction de masse de réactif dans chacune des marques de charbon, dans lequel la tension de surface $\gamma_{100}$, la tension de surface $\gamma_0$, un rapport de mélange de chacune des marques de charbon du mélange de charbon, et une fraction de masse d'un inerte et une fraction de masse d'un réactif dans chacune des marques de charbon sont déterminées en utilisant les équations et les procédés décrits dans la description ;

une détermination d'une corrélation entre la tension interfaciale et une force de coke produite à partir du mélange de charbon ;

une détermination, à partir de la corrélation, d'une tension interfaciale correspondant à une force souhaitée du coke ;

une détermination de fractions de masse des au moins deux marques de charbon de sorte que la tension interfaciale correspondant à une force souhaitée du coke ou une tension interfaciale inférieure est atteinte ; et une préparation d'un mélange de charbon en mélangeant ensemble les au moins deux marques de charbon dans les fractions de masse.

**3.** Procédé de préparation d'un mélange de charbon, le procédé consistant en un procédé pour une préparation d'un mélange de charbon qui inclut au moins deux marques de charbon, le procédé comprenant :

une détermination d'une tension de surface $\gamma_{100}$ et d'une tension de surface $\gamma_0$ de chacune des marques de charbon d'un mélange de charbon à partir d'une ligne de régression déterminée par le procédé pour une évaluation de charbon selon la revendication 1, la tension de surface $\gamma_{100}$ correspondant à une quantité inerte de 100 %, la surface de tension $\gamma_0$ correspondant à une quantité inerte de 0 % ;

une détermination de fractions de masse des au moins deux marques de charbon de sorte qu'une tension interfaciale de 0,26 mN/m ou moins, comme calculé à partir de la tension de surface $\gamma_{100}$, la tension de surface $\gamma_0$, un rapport de mélange de chacune des marques de charbon du mélange de charbon, et une fraction de masse d'inerte et une fraction de masse de réactif dans chacune des marques de charbon, est atteinte, dans lequel la tension de surface $\gamma_{100}$, la tension de surface $\gamma_0$, un rapport de mélange de chacune des marques de charbon du mélange de charbon, et une fraction de masse d'inerte et une fraction de masse de réactif dans chacune des marques de charbon, sont déterminées en utilisant les équations et les procédés décrits dans la description ; et une préparation d'un mélange de charbon en mélangeant ensemble les au moins deux marques de charbon dans les fractions de masse.

**4.** Procédé de préparation d'un mélange de charbon, le procédé consistant en un procédé pour une préparation d'un mélange de charbon qui inclut au moins deux marques de charbon, le procédé comprenant :

une détermination d'une tension de surface $\gamma_{100}$ et d'une tension de surface $\gamma_0$ de chacune des marques de charbon d'un mélange de charbon à partir d'une ligne de régression déterminée par le procédé pour une évaluation de charbon selon la revendication 1, la tension de surface $\gamma_{100}$ correspondant à une quantité inerte de 100 %, la surface de tension $\gamma_0$ correspondant à une quantité inerte de 0 %, dans lequel la tension de surface $\gamma_{100}$ et la tension de surface $\gamma_0$ sont déterminées en utilisant les équations et les procédés décrits dans la description ; et

une préparation d'un mélange de charbon en mélangeant ensemble les au moins deux marques de charbon de telle manière qu'une fraction de masse de charbon présentant une valeur absolue d'une différence entre la tension de surface $\gamma_{100}$ et la tension de surface $\gamma_0$ de 6 mN/m ou supérieure est inférieure ou égale à 45 % de masse dans le mélange de charbon.

**5.** Procédé de production de coke, le procédé comprenant :

une préparation d'un mélange de charbon en utilisant le procédé pour une préparation d'un mélange de charbon selon l'une quelconque des revendications 2 à 4 ; et
une production de coke par carbonisation du mélange de charbon.

## FIG. 1

## FIG. 2

## FIG. 3

## FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013145680 A

**Non-patent literature cited in the description**

- **NAGAYAMA MIKIYA et al.** Evaluation of Coal Compatibility Effect in Coke Strength by Surface Tension of Semi-coke. *ISIJ INTERNATIONAL*, 2017, vol. 57 (6), 989-995

- **D.W. FUERSTENAU**. *International Journal of Mineral Processing*, 1987, vol. 20, 153